# EUROPEAN PATENT APPLICATION

(11) **EP 2 028 260 A1**
(43) Date of publication of application: **25.02.2009**
(21) Application number: 07447047.7
(22) Date of filing: 01.08.2007
(51) Int. Cl.: C11C 3/02, C11C 3/10, C07C 67/00, C07C 67/03, C10L 1/02

(54) **Esterification process**

(71) Applicant: N.V. Desmet Ballestra Engineering S.A., 1935 Zaventem (BE)
(72) Inventor: De Greyt, Wim, 9112 Sinaai (BE); Kellens, Marc, 2812 Mechelen-Muizen (BE)
(74) Representative: Bird, William Edward

(57) **Abstract**

This invention provides a process for producing esters of fatty acids and one or more polyhydric alcohols comprising the steps of:
(a) providing a reaction mixture comprising free fatty acids and one or more polyhydric alcohols;
(b) heating said reaction mixture to a temperature at which said free fatty acids react with said one or more polyhydric alcohols ; and
(c) subjecting said reaction mixture to a flashing step generating a vapour stream and a flashing residue comprising said esters.

## Description

### FIELD OF THE INVENTION

The invention relates to a process for producing esters of fatty acids and one or more polyhydric alcohols that permits raw materials with a high (e.g. at least 10% by weight) free fatty acid content to be utilised for the production of biodiesel.

### BACKGROUND TO THE INVENTION

Fatty acid esters of lower alcohols such as, but not limited to, methanol, are used more and more as fuel in compression ignition (diesel) engines because they are renewable. Early ' biodiesel ' processes prescribed the use of neutral raw materials and thereby competed with food applications. Accordingly, there is an incentive to exploit cheaper alternative sources of fatty acid moieties as potential raw materials for biodiesel production. This often means that such materials may contain free fatty acids (hereinafter referred to as FFA) and that the FFA contents can vary over a wide range.

Examples of such alternative sources of fatty acid moieties are triglyceride oils with high free fatty acid contents. Their neutralisation would entail such high neutral oil losses that this treatment is no longer economically justified in the context of biodiesel production. Used frying oils fall into this category. Byproducts of the edible oil industry such as fatty acid distillates resulting from the physical refining of edible oils, and acid oils obtained by acidulating the soapstock resulting from the chemical neutralisation of edible oils are further examples of such alternative sources of fatty acid moieties.

Several biodiesel processes have been developed to cope with oils that are not neutral. U.S. Patent No. 2,383,601 discloses a process comprising treating a fatty glyceride containing free fatty acids with a low molecular weight monohydric alcohol in the presence of an acid esterification catalyst, thereafter reacting said fatty glyceride with said low molecular weight monohydric alcohol in the presence of sufficient alkaline agent to neutralise any acid present and to provide an alkaline alcoholysis catalyst whereby a liquid body containing an ester of fatty acid from said glyceride with said alcohol and glycerine is produced, and recovering said ester and glycerine from said liquid body.

U.S. Patent No. 5,434,279 discloses a process that is characterised in that non-purified fatty acid glycerides, containing in particular high proportions of free fatty acids and phosphatides are esterified in one or several steps in the presence of a basic catalyst with an excess of short-chain alcohol. In this process, the basic catalyst will react with the free fatty acids present in the non-purified fatty acid glycerides. Consequently, the catalyst requirement is high and the free fatty acids are not converted into biodiesel.

Therefore, other processes have been developed that aim at utilising free fatty acids as raw material for the production of biodiesel. One such process, as disclosed in U.S. Patent No. 5,849,939, recuperates the fatty acids moieties from the soaps formed when a basic catalyst reacts with the free fatty acids present in the raw material by acidifying the glycerol phase resulting from the methanolysis of said raw material. This recuperation entails the esterification of these fatty acids with methanol while using an acid catalyst. The resulting esterification product can then be recycled to a different reaction mixture comprising triglyceride oil and for instance methanol.

The free fatty acids present in a fatty raw material can also be esterified, with for instance methanol, before said material is transesterified with methanol to form fatty acid methyl esters as disclosed in U.S. Patent No. 6,696,583 wherein a fatty acid phase is mixed with a further mixture of triglycerides and fatty acids and the fatty acids contained in the mixture thus obtained are esterified with an alcohol, whereby an esterification mixture containing triglycerides and fatty acid alkyl esters is obtained, which esterification mixture is transesterified with alcohol so as to form further fatty acid alkyl esters.

The esterification steps mentioned in the above patents involve the use of a catalyst. Preferred embodiments prescribe the use of acid catalysts such as sulphuric acid. However, such acids have the disadvantage that they may catalyse other reactions such as the condensation of glycerol moieties. Moreover, said acids need to be neutralised and the resulting neutralisation products need to be removed from the reaction medium. These additional process steps dramatically increase the processing and manufacturing cost of biodiesel.

Accordingly, there is a clear need for a process that allows fatty raw materials with variable free fatty acid contents to be effectively utilised as fatty feed for biodiesel production.

### OBJECT OF THE INVENTION

Accordingly, it is an object of the present invention relates to processes for utilising fatty raw materials with a high (e.g. at least 10% by weight) free fatty acid content for the production of fatty acid esters of lower alkyl monohydric alcohols.

### SUMMARY OF THE INVENTION

It has surprisingly been found that the above object can be efficiently met by a process for producing esters of fatty acids and one or more polyhydric alcohols, comprising the steps of:
a) providing a reaction mixture comprising free fatty acids and one or more polyhydric alcohols;
b) heating said reaction mixture to a temperature at which said free fatty acids react with said one or more polyhydric alcohols; and
c) subjecting said reaction mixture to a flashing step generating a vapour stream and a flashing residue comprising said esters.

The process according to any embodiment of the present invention can advantageously be used when converting a fatty feed (such as hereinafter defined) into lower alkyl esters of fatty acids to be used as biodiesel or biofuel. This may be performed by converting a raw material containing free fatty acids into a starting material for a transesterification process, said starting material having a low (e.g. about 0.5% by weight or less) residual free fatty acid content and thus requires only the minimal amount of transesterification catalyst. Moreover, certain embodiments of the process according to the invention are able to maximise the yield of lower alkyl esters of fatty acids.

It is an advantage of the present invention to minimise and even avoid the use of esterification catalysts in step (b) and consequently the need for their subsequent neutralisation and/or removal.

It is another advantage of the present invention to provide a process for the production of esters of fatty acids and one or more polyhydric alcohols that can be operated continuously.

It is a further advantage of the present invention to enable the maximisation of the yield of lower alkyl esters of fatty acids based on the fatty acid moiety content of the raw material.

It is yet another advantage of the present invention to provide a process that can accommodate a wide range of raw materials with varying free fatty acid contents.

The present invention also provides biodiesel having lower alkyl esters of fatty acids converted from a fatty feed (such as hereinafter defined). This conversion may be performed by converting a raw material containing free fatty acids into a starting material for a transesterification process, said starting material having a low (e.g. about 0.5% by weight or less) residual free fatty acid content and thus requires only the minimal amount of transesterification catalyst.

These and further advantages of the present invention will become apparent from the description and the examples hereinafter.

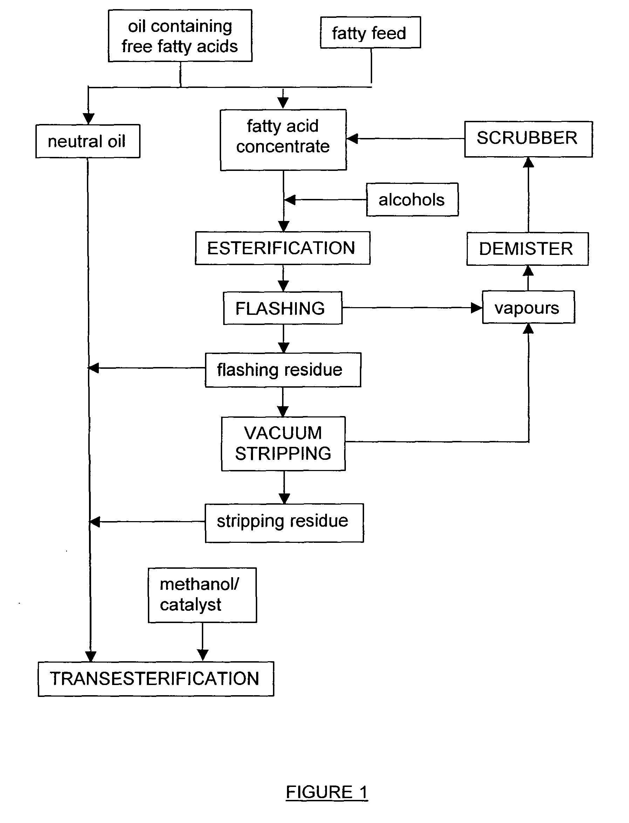

### BRIEF DESCRIPTION OF THE DRAWING

The single figure represents a flow diagram of the process according to one illustrative embodiment of the present invention.

### DEFINITION OF TERMS

The terms defined below are shown in capitals and have been listed alphabetically; if a definition contains a listed term, this term has been italicised.
■ ACID OILS is the fatty product that results from the *acidulation* of *soapstock.* Its composition varies but it is likely to contain more than 50 % by weight of free fatty acids, the remainder comprising triglyceride oil, *partial glycerides* and unsaponifiables.
■ ACIDULATION is the process used to recover the fatty matter contained in product streams comprising soaps. It involves adding an acid as for instance sulphuric acid, to this product stream and separating the fatty phase as *acid oils* from the aqueous phase.
■ ALCOHOLYSIS is the reaction between an alcohol and a glyceride such as an oil or fat. If the alcohol concerned is methanol, the *alcoholysis* can also be referred to as 'methanolysis' and if it is glycerol, the term 'glycerolysis' can be used;
■ CRUDE OIL is the general name for an oil or fat as isolated from its source and that has not undergone any treatment except perhaps a water degumming treatment ensuring that the crude oil meets trading specifications and does not throw a deposit during storage and transport. Crude oil therefore may contain free fatty acids and/or gums.
■ DIGLYCERIDES is the trivial name of diacyl glycerides, molecules in which two acyl moieties are linked to the same glycerol molecule through ester bonds, leaving one free hydroxyl group.
■ ESTERIFICATION is the formation of an ester from an alcohol and an acid such as a fatty acid, whereby water is formed at the same time. This formation can be catalysed by an acid such as sulphuric acid or by an enzyme like lipase but at sufficiently high temperature, no catalyst is needed for the esterification to proceed.
■ FAME is the abbreviation of Fatty Acid Methyl Esters.
■ FATTY ACID DISTILLATE refers to the *scrubber condensate* in the case of the physical refining process
■ FATTY FEED is a general description of a raw material comprising triglyceride oil, *partial glycerides* and/or *FFA.*
■ FFA is the standard abbreviation of Free Fatty Acids
■ FLASHING is a process step in which the pressure at which a mixture is held is suddenly lowered causing some of the most volatile constituents of said mixture to evaporate.
■ HYDROLYSIS is the reverse reaction of the *esterification* and in practice these reactions lead to equilibria. A high degree of hydrolysis therefore results from increasing the water concentration, whereas low concentrations of free acids and/or free alcohols can be induced by water removal.
■ MONOGLYCERIDES is the trivial name of monoacyl glycerides, molecules in which one acyl moiety is linked to a glycerol molecule through an ester bond.
■ PARTIAL GLYCERIDES are partially hydrolysed triglycerides. Diacyl glycerol, having two acyl groups and one free hydroxyl group is commonly referred to as a *diglyceride.* A *monoglyceride* has only one acyl group left and thus has two free hydroxyl groups.
■ SCRUBBER CONDENSATE is the product that arises when the organic compounds present in the vapours extracted from the deodorisation vessel are condensed. In the case of physical refining, the fatty acid content of the scrubber condensate is quite high so it is then also referred to as *fatty acid distillate*
■ SOAPSTOCK is the by-product of the chemical neutralisation of crude triglyceride oils. It comprises soaps, phosphatides and neutral oil besides many colouring compounds, particulate matter and other impurities as well as water containing various salts.
■ SODIUM METHYLATE is a trivial name for sodium methanolate, a base that catalyses *alcoholysis.*
■ TRANSESTERIFICATION is another name for *alcoholysis.*

### DETAILED DESCRIPTION OF THE INVENTION

The present invention will be described with respect to particular embodiments, and with reference to the single attached drawing but the invention is not limited thereto but only by the claims.

The methanolysis reaction used to produce FAME from a fatty feed is commonly catalysed by a base such as an alkali hydroxide or alkoxide, in particular sodium hydroxide or sodium methylate. This catalyst reacts with any FFA present in the reaction mixture with formation of soaps and, in order to maintain catalytic activity, the amount of catalyst must exceed the FFA content on a molar basis. Soaps formed during this transesterification reaction dissolve in the glycerol layer formed during the methanolysis reaction but they tend to retard the separation of this layer by stabilising the emulsion. Once the glycerol layer has been separated, the fatty acid moieties present as soaps can be recuperated by acidulating the glycerol and separating the fatty acid layer from the heavier glycerol phase. The fatty acids thus obtained can serve as fatty feed for the process of the present invention.

Various waste products like used frying oil, fatty matter from grease traps, inedible tallow or other waste fats can also be profitably used in the process of the invention.

Other sources of fatty feeds with a high FFA content are for instance the scrubber condensate as obtained during the physical refining of edible oils. In this process, edible oils or fats containing some FFA are subjected to a vacuum stripping process at a temperature up to 260°C. The most commonly used stripping medium is steam and a commonly used operating pressure is from about 2 to 10 hPa. The vapours leaving the oil are generally passed through a vapour scrubber where most organic compounds present in the vapour stream condense. The resulting condensate has a high FFA content which can be in excess of 90 % by weight if the oil being subjected to the vacuum stripping process already has a significant FFA content and the stripping process is intended for neutralising this oil. If on the other hand, the oil has already been neutralised by a treatment with alkali, the scrubber condensate has a lower FFA content, which can be below about 20 % by weight. Even so, the latter condensate can also serve as a fatty feed for the process of the present invention. Similarly, the acid oils resulting from the acidulation of soaps formed and isolated during the alkali refining of edible oils can also serve as a fatty feed for the process of the present invention.

The process of the present invention is capable of reducing the FFA content of crude oils, which may be from about 2 to 5 %, expressed as oleic acid. However, for cost reasons it may be preferred to concentrate the FFA present in the oil by neutralising the oil either physically by steam stripping or chemically with alkali, and then operate the process of the present invention on the FFA concentrate. The higher the FFA concentration in the reaction mixture of the process of the present invention, the higher the reaction rate and thus a higher productivity calculated on a reactor volume basis.

This feature is especially advantageous when the process of the present invention is operated in symbiosis with a transesterification process producing biodiesel. Neutralisation of a fatty feed comprising FFA provides this transesterification process with a neutral feed requiring a minimum of base catalyst and producing a minimum of soaps. Moreover, the by-product glycerol resulting from this transesterification process can be used in step (a) of the process of the present invention as a source of the required polyhydric alcohol.

Suitable polyhydric alcohols for the performance of the present invention can be triols like glycerol (1,2,3-propanetriol) or diols like ethylene glycol (1,2-ethanediol) or propylene glycol (1,2-propanediol). The use of glycerol as the required polyhydric alcohol has the additional advantage that it utilises a by-product of the methanolysis reaction used to produce biodiesel. Since this methanolysis reaction provides a polyhydric alcohol to be used in the process of the present invention, at least part of this polyhydric alcohol can be recycled.

The main criteria that a polyhydric alcohol has to meet are in order to be suitable in the process of the present invention are:
- it has a higher boiling point than water so that fractionation is possible and the esterification equilibrium can be shifted away from the FFA side; the desirable difference in boiling points between water and the polyhydric alcohol depends, in a manner well known to the skilled person, upon factors such as but not limited to the length of the fractionation column and the alcohol content of the water that has been removed by distillation to shift the esterification equilibrium toward the ester side; and
- it remains chemically stable in the presence of acids at the esterification temperature reached in step (b) of the process of the invention.

The quality requirements of the one or more polyhydric alcohols to be provided in step (a) of the process of the invention are not strict. The one or more polyhydric alcohols are preferably dry but, since water is formed when said one or more polyhydric alcohols are esterified with free fatty acids in step (b) of the process according to the invention, any residual water present in the starting one or more polyhydric alcohols will only affect the position of the esterification equilibrium before the flashing step (c). During the flashing step (c) of the process according to the present invention, any water present in the reaction mixture will evaporate and this will cause the position of the equilibrium to shift if the reaction mixture is still reactive.

The molar ratio of the one or more polyhydric alcohols and the free fatty acids in the reaction mixture provided in step (a) of the process of the invention is not critical. The molar ratio of the hydroxyl groups present in the one or more polyhydric alcohols and the carboxyl groups present in the free fatty acids preferably ranges from about 1.5:1 to 6:1, more preferably (2:1 to 5:1), most preferably (3:1 to 4:1). Such ratios advantageously lead to high reaction rates and/or prevent a large excess of one of the reagents having to be flashed off in step (c) of the process of the present invention.

In step (b) of the process of the invention, the reaction mixture provided in step (a) is heated to a temperature at which the one or more polyhydric alcohols and the free fatty acids react by forming ester bonds. In practice, this means a reaction temperature of at least 200°C and up to 280°C when no catalyst is present. In the presence of an esterification catalyst, a reaction temperature below 200°C may ordinarily be sufficient. In the latter situation, a suitable reaction temperature depends, in a manner known to the person skilled in the art, upon different parameters such as the efficacy, type and concentration of the catalyst. A higher reaction temperature has the advantage of increasing the reaction rate but the disadvantage of correspondingly increasing the reaction pressure. The reaction temperature may also be limited by the undesirable occurrence of side reactions such as, for example, a polycondensation of the one or more polyhydric alcohols.

The use of a catalyst in the esterification in step (b) of the process of the invention is optional. Catalysts that can be used in the process of the invention can be homogeneous or heterogeneous in type. Heterogeneous catalysts such as for instance acid activated bleaching earth, can be in suspension or in the form of a packed bed. Packed bed catalysts have the advantage that they do not require a removal step but have the disadvantage that they no longer provide catalytic activity after the flashing stage (c) and cannot catalyse the shift of the esterification equilibrium towards the ester side after the free water has been flashed off.

A homogeneous esterification catalyst that is cheap, effective and well proven is a strong inorganic acid such as sulphuric acid. Its main disadvantages however are:
- at high temperature, it can cause charring;
- when removed by water washing, the diluted acid is highly corrosive for the installation wherein the process is performed, and
- it is hygroscopic and thus may impede the removal of water during the flashing step (c) of the process.

Strong organic acids, e.g. p-toluene sulphonic acid, do not suffer from several of these disadvantages but are usually more expensive.

A particulate esterification catalyst that has been found to be highly effective is acid activated bleaching earth. When used in amounts of about 1 to 2 % by weight, this type of particulate catalyst is quite effective while decreasing the reaction temperature to around 170°C and it can easily be removed from the reaction mixture by filtration. It may cause some double bonds present in the fatty feed to isomerise geometrically, *i*.*e*. from the *cis* configuration to the *trans* configuration. This consequently raises the melting point of compounds incorporating the fatty acid moiety, such as FAME. The heterogeneous stationary catalysts that may be used in the process of the invention are also acid in character and preferably inorganic in order to withstand the reaction temperature.

In one embodiment, the process of the invention is carried out continuously. This means that the reaction mixture provided in step (a) is fed, e.g. by a positive pump, into a reactor that preferably has a plug flow design. Because of the pressure build up when water is formed, the reactor should preferably be able to withstand an operating pressure of up to about 2.5 MPa. Accordingly, a long coil immersed in a heat exchange medium such as Dowtherm® constitutes an appropriate reactor design option but the process of the present invention is in no way limited to this design. Satisfactory results have also been obtained with agitated tank reactors.

In step (b) of the process according to the present invention, the pressure inside these reactors may have to be controlled. This can conveniently be done by using a pressure control valve that is connected to a condensing system. If the pressure is controlled at a sub-atmospheric value, the condensing system must be connected to a vacuum system. Operating under vacuum (preferably above about 10 hPa absolute) has the advantage that water is gradually removed from the reaction mixture as and when it is formed by the esterification reaction. Consequently the esterification equilibrium is shifted towards the ester side, thus in turn causing the productivity of the process to increase. On the other hand, the vacuum should not be so deep, for instance the pressure during step (b) shoud not be below 10 hPa absolute, as not to permit subsequent flashing in step (c) of the process according to the invention. A too deep vacuum may also cause the one or more polyhydric alcohols to evaporate and be removed from the reaction mixture, although a small fractionating column before the pressure control valve can assist in retaining this polyhydric alcohol in the reaction mixture.

Because the reaction products increase the solubility of the one or more polyhydric alcohols in the fatty acid phase, recycling a part, for instance from about 10 to 40% by weight, of the reaction product to the reaction mixture being fed to the reactor can assist in getting the reaction in a plug flow reactor underway.

The rate of esterification of the fatty acids and the one or more polyhydric alcohols is proportional to the concentrations of their respective functional groups. For instance, if equimolar amounts of free fatty acids and glycerol are allowed to react together, the esterification rate decreases to 42% of its original value when half of the free fatty acids have reacted. With a smaller excess of hydroxyl groups, the esterification rate decrease is even larger. In addition, taking into account that the esterification reaction is reversible and an equilibrium will be reached, the reaction rate decreases even more. Consequently, in one embodiment of the process of the present invention, the esterification reaction of step (b) is stopped well before equilibrium of the esterification reaction is reached. In one embodiment of the process of the present invention, step (c) is started as soon as the amount of ester formation in step (b) has reached a predetermined level.

In the continuous embodiment of the process of the present invention, a pressure control valve is preferably fitted at the end of the reactor coil. This pressure control valve may be connected to a flash vessel kept at a lower pressure than the reactor. The pressure inside the flash vessel can be above atmospheric pressure but is preferably below atmospheric pressure, for instance but not limited to the range between 3 and 50 hPa. In the latter situation, the flash vessel can be connected to a vacuum system, e.g. via a demister and a scrubber, as schematically indicated in the single figure.

At the bottom of the flash vessel, the reaction product having lost part of, e.g. most of, its volatile content may be collected in a sump, from where a pump can extract it for further processing or storage. The residual FFA content of this material depends on a number of factors, including the FFA content of the reaction mixture provided in step (a). The higher this content is high, the larger the amount of water formed during esterification, and the more effective the entrainment of residual FFA during the flashing step (c) of the process according to the invention.

The pressure in the flash vessel is another of such factors. If such pressure is low, water being flashed off occupies a larger volume and therefore entrains more free fatty acids. Similarly, a higher temperature will cause more free fatty acids to be entrained just as in physical refining or deodorisation processes.

Water is the most volatile constituent of the reaction mixture but the one or more polyhydric alcohols also evaporate when exposed to the reduced pressure in the flashing vessel. The gas volume thus created entrains free fatty acids and even some monoesters of the one or more polyhydric alcohols and fatty acid formed during the esterification step (b). Their vapour pressure as pure compounds is higher than that of fatty acids, which decreases their volatility, but their molar concentration is higher especially after most fatty acids have flashed off, and this increases their partial vapour pressure.

Suddenly flashing off all volatile components simultaneously in step (c) may cause non-volatile components to be finely dispersed as a mist that is sucked away by the vacuum system without being retained by the demister. Consequently, it can sometimes be advantageous to only partially remove the volatile components in the flashing step (c) and to rely on a further vacuum stripping process for additional volatile removal. This vacuum stripping process can be performed in a separate vessel as indicated in the single figure, but the vessel in which the flashing step (c) is performed can also be constructed in such a way that it also serves as a vacuum stripping vessel. In the latter instance, the vessel may comprise a number of superimposed trays that allow the reaction product to move downwards from one tray to the next tray. Heating the material while it moves downwards and sparging the trays with a stripping medium will cause a more gradual volatile removal than sudden flashing off. In addition, the vessel can be provided with a bottom tray having a heat exchange coil that allows the stripping residue to be cooled by the reaction mixture provided in step (a) of the process of the invention.

By controlling the temperature of the reaction mixture before it enters the flashing vessel and especially by controlling the pressure inside this vessel, the residual FFA content of the reaction product can be effectively controlled. If the reaction product is mixed with neutral oil before being transesterified, a resulting residual FFA content of about 2% by weight is normally fully acceptable from an industrial and economical standpoint. If the reaction product is transesterified on its own, a residual FFA content lower than 2% by weight should preferably be aimed for.

In another embodiment of the process of the invention, the vapours leaving the flashing vessel and the stripping vessel are passed to a scrubber where all organic components (one or more polyhydric alcohols, free fatty acids, and some monoglycerides if the one or more polyhydric alcohols contain glycerol) will almost completely condense. The condensate thus obtained is preferably recycled to the reaction mixture provided in step (a) of the process of the invention; in the single attached figure, this has been indicated by the arrow connecting the ' scrubber ' to the ' fatty acid concentrate '.

The single attached figure also shows a non-limiting embodiment wherein the flashing residue and/or the stripping residue are transesterified with methanol either on their own or after having been mixed with neutral triglyceride oil.

The following examples are provided only for the purpose of illustration of the invention and should not be understood as limiting the invention in any aspect.

### COMPARATIVE EXAMPLE 1

In this example, a fatty acid distillate resulting from the physical refining of vegetable oils was used as a starting material. Its FFA content was 88.9%, expressed as oleic acid. In addition, this fatty acid distillate contained small amounts of monoglycerides, diglycerides and triglycerides. About 400 g of this starting material was mixed with 133 g glycerol (99.5% purity) to form an almost equimolar reaction mixture of free fatty acids and glycerol which was pre-heated to 80°C.

The pre-heated mixture was placed in a 1 litre agitated PARR reactor and heated to the reaction temperature of 200°C while the agitation was set at its maximum value. After a reaction time indicated in Table 1, the mixture was allowed to cool and, when the temperature had reached about 80°C, the reactor was vented and its contents were transferred to a separating funnel and the FFA content expressed as oleic acid of the upper layer resulting from separation was analytically determined. Because heating and cooling are not instantaneous, a reaction time of zero minute also leads to some esterification, as shown by Table 1 below:

**Table 1**

| | | | | |
|---|---|---|---|---|
| Reaction time (minutes) | 0 | 60 | 180 | 300 |
| FFA content upper layer (%) | 78.9 | 41.7 | 19.4 | 11.8 |

Table 1 clearly shows that the rate of esterification slows down quite rapidly: during the first hour of reaction more ester bonds were formed than during the next two hours and continuing the reaction for a further two hours caused only a slight increase in conversion. However, even after five hours the residual FFA content of the reaction mixture was still so high that transesterification of this reaction mixture will necessarily entail significant losses of fatty acid moieties.

### COMPARATIVE EXAMPLE 2

The same starting materials were used as in Example 1 and in the same ratio but instead of being carried out under its autogenous pressure as in Example 1, the esterification reaction was carried out under reduced pressure in a Rotavapor apparatus kept at a pressure of 330 hPa absolute. As shown in Table 2, reducing this pressure significantly increased the reaction rate with respect to the corresponding values of table 1:

**Table 2**

| | | | |
|---|---|---|---|
| Reaction time (minutes) | 60 | 180 | 300 |
| FFA content upper layer (%) | 34.2 | 13.4 | 7.5 |

However, even after 300 minutes, the residual FFA content was still unacceptably high for further processing of the resulting reaction mixture by transesterification.

### EXAMPLE 3

In order to further significantly reduce, with respect to examples 1 and 2, the residual FFA content of the esterification reaction product to acceptably low levels, the process of the invention was applied in a deodorisation unit comprising a flask fitted with a sparge ring with 1 mm holes near the bottom of the flask, which flask was connected to a vacuum system via a condenser. The sparge ring was provided with water vapour generated in the oven in which the flask has been positioned; this set-up prevents vapours generated during the flashing operation from condensing onto the upper walls of the flask and returning to its contents.

The flashing step of this experimental process was carried out by placing the reaction mixture resulting from the esterification reaction of Example 1 after 300 minutes into the deodorisation flask, and setting the oven temperature at 200°C. The vacuum was maintained at 10 hPa absolute and a small amount of steam was passed through the reaction mixture to provide some agitation. When the temperature of 200°C had been reached, the pressure was reduced to 3 hPa and a timer was started. Lowering the pressure caused the mixture to boil vigorously. The vapours were condensed and the total condensate was analysed.

After 30 minutes distillation, the oven door was opened in order to allow the flask and its contents to cool down. When their temperature had decreased to 70°C, the apparatus was switched off and the contents of the deodorisation flask were analysed for all components, including the FFA content expressed as oleic acid.

**Table 3**

| | Starting material | Distillate | Distillation residue |
|---|---|---|---|
| Yield (%) | 100.0 | 12.5 | 86.5 |
| FFA content (%) | 11.8 | 25.6 | 3.2 |
| Glycerol (%) | 3.7 | 31.0 | 0.09 |
| Monoglycerides (%) | 31.6 | 11.8 | 24.4 |
| Diglycerides (%) | 28.8 | < 0.1 | 40.0 |
| Triglycerides (%) | 6.3 | < 0.1 | 12.8 |

Table 3 shows that the distillate contained the most volatile components of the starting material and very minor amounts of di- or triglycerides. It also shows that this distillate, in view of its composition, can suitably be recycled to an esterification reaction. Table 3 also shows that upon performance of the flashing step, further di- and triglycerides are formed at the expense of free fatty acids and of monoglycerides present in the starting material. Accordingly, the flashing operation of the process according to the invention causes less glycerol to be formed during a transesterification reaction, and achieves a residual FFA content of the flashing residue which is low enough for avoiding significant losses of fatty acid moieties in a transesterification of said residue.

## Claims

1. A process for producing esters of fatty acids and one or more polyhydric alcohols comprising the steps of:
(a) providing a reaction mixture comprising free fatty acids and one or more polyhydric alcohols;
(b) heating said reaction mixture to a temperature at which said free fatty acids react with said one or more polyhydric alcohols ; and
(c) subjecting said reaction mixture to a flashing step generating a vapour stream and a flashing residue comprising said esters.

2. A process according to claim 1, in which the vapour stream flashed off in step (c) is partially condensed and the condensate thus obtained is at least partially recycled into the reaction mixture provided in step (a).

3. A process according to claim 1 or claim 2, in which the flashing residue generated in step (c) is subjected to a vacuum stripping process, thus providing a stripping vapour stream and a stripping residue.

4. A process according to claim 3, in which said stripping vapour stream is partially condensed and the condensate thus obtained is at least partially recycled into the reaction mixture provided in step (a).

5. A process according to claims 1 or 2, in which said flashing residue is at least partially used as starting material for the transesterification with a C₁₋₄ alkyl monohydric alcohol in the presence or absence of a neutral triglyceride oil.

6. A process according to claim 3 or claim 4, in which the stripping residue is at least partially used as starting material for the transesterification with a C₁₋₄ alkyl monohydric alcohol in the presence or absence of a neutral triglyceride oil.

7. A process according to any of claims 1 to 6, in which said one or more polyhydric alcohol comprises glycerol.

8. A process according to claim 5 or claim 6 in which the lower alkyl monohydric alcohol is methanol.

9. A process according to any of the claims 1 to 8, in which the molar ratio between the hydroxyl groups in said one or more polyhydric alcohols and the free fatty acids ranges from 1.5:1 to 6:1.

10. A process according to any of claims 1 to 9, in which from 10 to 40% by weight of the flashing residue is incorporated into the reaction mixture provided in step (a).

11. A process according to any of claims 1 to 10, in which the esterification reaction in step (b) is catalysed by an acid or an acid activated bleaching earth.

12. A process according to any of claims 1 to 10, in which the esterification reaction in step (b) is performed in the absence of a catalyst.

13. A process according to claim 11 in which the acid catalyst is immobilised as a packed bed.

14. A process according to any of claims 1 to 13, in which the pressure during step (b) is controlled at a value above 10 hPa absolute and below 0.5 Mpa gauge.

15. A process according to any of claims 1 to 14, in which the vapour stream generated in step (c) is subjected to fractionation.

16. A process according to any of claims 1 to 15, wherein said flashing residue contains said esters and no more than 5% by weight free fatty acids.

17. A process according to any of the previous claims, wherein said esters are utilised in the production of biodiesel.
